# EUROPEAN PATENT APPLICATION

(11) **EP 1 942 087 A1**
(43) Date of publication of application: **09.07.2008**
(21) Application number: 06811286.1
(22) Date of filing: 05.10.2006
(51) Int. Cl.: C07B 35/06, C07B 61/00, C07C 1/30, C07C 15/04, C07C 15/58, C07C 35/06, C07C 67/317, C07C 69/74

(54) **METHOD FOR DEHYDROHALOGENATION OF ORGANIC HALOGEN COMPOUND**

(30) Priority: 05.10.2005 JP 2005292090
(71) Applicant: Daiichi Sankyo Company, Limited, Chuo-ku Tokyo 103-0023 (JP)
(72) Inventor: SATO, Koji, Tokyo 1348630 (JP); IMAI, Makoto, Tokyo 1348630 (JP)
(74) Representative: Hartz, Nikolai
(86) International application number: PCT/JP2006/319948
(87) International publication number: WO 2007/040259

(57) **Abstract**

The present invention relates to a hydrodehalogenation method in which a halogen atom on a carbon atom of an organic compound is removed by substituting the halogen atom with a hydrogen atom, and to a method for producing a dehalogenated compound. The hydrodehalogenation method is **characterized by** including treating an organic compound having a halogen atom on a carbon atom thereof in a solvent with a compound represented by formula (2-1):

M²BHₚR¹_{q} (2-1)

or formula (2-2) :

M³(BHₚR¹_{q})₂ (2-2)

(wherein M² represents an alkali metal atom; M³ represents an alkaline earth metal atom or a zinc atom; R¹ represents a hydrogen atom, a cyano group, a C2-C13 acyloxy group, or a C1-C6 alkoxy group; p is an integer of 1 to 4; q is an integer of 0 to 3; and the sum of p and q is 4) in the presence of a Group VIII metal complex represented by formula (1):

M¹XₘLₙ (1)

(wherein M¹ represents a Group VIII metal; X represents a halogen atom; L represents a neutral ligand; m is an integer of 1 or 2; n is an integer of 2 or 3; and the sum of m and n is 4).

## Description

### Technical Field

The present invention relates to a hydrodehalogenation method in which a halogen atom on a carbon atom of an organic compound is removed by substituting the halogen atom with a hydrogen atom, and to a method for producing a dehalogenated compound.

### Background Art

Organic halogen compounds having a halogen atom such as chlorine are generally harmful to living organisms as exemplified by PCBs and dioxins. These compounds are stable and hardly degradable in the natural environment, therefore, if they are once released to the environment, these compounds are accumulated to contaminate the environment. Even such harmful organic halogen compounds, they can be rendered harmless or low-toxic through removal of halogen in the compounds (i.e., dehalogenation). Such dehalogenation methods include chemical extraction and decomposition, decomposition with metallic sodium, hydroxylation with supercritical water, and also, incineration is carried out for rendering these compounds harmless or non-toxic. However, since these dehalogenation techniques require considerably severe reaction conditions, cost for plants and apparatus is disadvantageously high.

Meanwhile, in organic chemistry, dehalogenation of organic halogen compounds is widey used, and hitherto, a variety of methods and reagents for dehalogenation have been actually developed. However, as for the aromatic chlorine and aromatic fluorine compounds, no simple dehalogenation method has been provided. In recent years, there have been reported hydrodechlorination methods for aromatic chlorine compounds; for example, reaction with Et₃SiH in the presence of PdCl₂ (see Non-Patent Document 1), reaction with Et₃SiH in the presence of RhCl(PPh₃)₃ (Wilkinson's complex) (see Non-Patent Document 2), and reaction with a Grignard reagent in the presence of FeCl₃ (see Non-Patent Document 3). However, these methods are not satisfactory from the industrial view point. For example, since reagents employed in these methods exhibit high reactivity, but in case of Et₃SiH, this has a boiling point of 107 to 108°C and hardly to be separated from a product of a boiling point. In addition, such reagents themselves are very expensive, and a Grignard reagent must be handled under anhydrous conditions, which is cumbersome. There has also been reported hydrodefluorination of aromatic fluorine compounds including reaction with a Grignard reagent in the presence of CpTiCl₃ (see Patent Document 1). This method is not an industrially satisfactory method, since the method employs the aforementioned Grignard reagent, which must be handled under anhydrous conditions.
Patent Document 1: JP-A-2005-82539
Non-Patent Document 1: R. Boukherroub et al., Organometallics, 15, 1508, 1996
Non-Patent Document 2: Miguel A. Esteruelas et al., Organometallics, 18, 1110, 1999
Non-Patent Document 3: H. Guo et al., Chemistry Letter, 33, 10, 1356, 2004

### Disclosure of the Invention

### Problems to be Solved by the Invention

An object of the present invention is to provide a hydrodehalogenation method which employs a reagent that allows scaling-up of hydrodehalogenation to an industrial level and which realizes simple and effective removal of a halogen atom from an organic halogen compound by substituting the halogen atom with a hydrogen atom. Means for Solving the Problems

The present inventors have carried out extensive studies on hydrodehalogenation reaction of organic halogen compounds, and have found that, through treating an organic halogen compound in the presence of a catalytic amount of a Group VIII metal complex and a hydrogen source, a dehalogenated compound can be produced at high yield, and that a reducing reagent, which is inexpensive and allows easy handling, can be employed as a hydrogen source. The present invention has been accomplished on the basis of these findings.

[0006] to [0012] Accordingly, the present invention is directed to a hydrodehalogenation method including treating an organic compound having a halogen atom on a carbon atom thereof in a solvent with a compound represented by formula (2-1):

M²BHₚ R¹_{q} (2-1)

or formula (2-2):

M³ (BHₚ R¹_{q}) ₂ (2-2)

(wherein M² represents an alkali metal atom; M³ represents an alkaline earth metal atom or a zinc atom; R¹ represents a hydrogen atom, a cyano group, a C2-C13 acyloxy group, or a C1-C6 alkoxy group; p is an integer of 1 to 4; q is an integer of 0 to 3; and the sum of p and q is 4) in the presence of a Group VIII metal complex represented by formula (1):

M¹XₘLₙ (1)

(wherein M¹ represents a Group VIII metal; X represents a halogen atom; L represents a neutral ligand; m is an integer of 1 or 2; n is an integer of 2 or 3; and the sum of m and n is 4).

[0013] to [0019] The present invention is also directed to a method for producing a dehalogenated compound, characterized by comprising treating an organic compound having a halogen atom on a carbon atom thereof in a solvent with a compound represented by formula (2-1):

M²BHₚ R¹_{q} (2-1)

or formula (2-2):

M³ (BHₚ R¹_{q}) ₂ (2-2)

(wherein M² represents an alkali metal atom; M³ represents an alkaline earth metal atom or a zinc atom; R¹ represents a hydrogen atom, a cyano group, a C2-C13 acyloxy group, or a C1-C6 alkoxy group; p is an integer of 1 to 4; q is an integer of 0 to 3; and the sum of p and q is 4) in the presence of a Group VIII metal complex represented by formula (1):

M¹XₘLₙ (1)

(wherein M¹ represents a Group VIII metal; X represents a halogen atom; L represents a neutral ligand; m is an integer of 1 or 2; n is an integer of 2 or 3; and the sum of m and n is 4).

### Effects of the Invention

The hydrodehalogenation method according to the present invention, employing a metal borohydride compound as a hydrogen source, has been realized as industrially applicable, which is difficult to be accomplished through a previous method from the viewpoints of cost and operability. Furthermore, the present invention is remarkably advantageous, since hydrodehalogenation of an organic halogen compound such as an aromatic halogen compound (e.g., an endocrine-disrupting chemical) which is harmful and hardly degradable can be completed under relatively mild conditions. Best Modes for Carrying Out the Invention

The Group VIII metal complex employed in the present invention is a compound represented by formula (1):

M¹XₘLₙ (1)

(hereinafter referred to as "Compound (1)").
In formula (1), M¹ represents a Group VIII metal such as rhodium, palladium, ruthenium, platinum, cobalt, or nickel. Among these, rhodium and palladium are preferred, with rhodium being particularly preferred.

Examples of the halogen atom represented by X include fluorine, chlorine, bromine, and iodine.

L represents a neutral ligand. No particular limitation is imposed on the species of the neutral ligand, so long as they can serve adjusting the reactivity of the metal.
Specific examples include phosphine, trialkylamine, nitrile, isonitrile, diene, arene, carbonyl, carbene, alkene, alkyne, cyclobutadiene, cycloheptatriene ether, olefin, and thioether. In some cases, a reaction substrate itself conceivably plays a role of ligand. Among these, phosphine (PR²R³R⁴) are preferably employed. In the line formula, R², R³, and R⁴, which may be the same or different, each represents an aliphatic (hydrocarbon) group or aromatic (hydrocarbon) group. Examples of the aromatic group include C6-C14 aromatic hydrocarbon groups such as phenyl and naphthyl, and C6-C14 aromatic hydrocarbonoxy groups such as phenoxy. These groups may be substituted by one or more ordinary employed functional groups. Examples of the aliphatic group include C1-C30 alkyl groups and C1-C30 alkoxy groups, which may be linear, branched, or cyclic. Specific examples include methyl, ethyl, propyl, isopropyl, tert-butyl, pentyl, hexyl, methoxy, ethoxy, n-propoxy, isopropoxy, n-butoxy, isobutoxy, sec-butoxy, tert-butoxy, and cyclopentyloxy.

Examples of the phosphine ligand include tertiary phosphines such as trimethylphosphine, triethylphosphine, tripropylphosphine, tributylphosphine, tricyclohexylphosphine, triphenylphosphine, dimethylphenylphosphine, diphenylmethylphosphine, 2-di-tert-butylphosphino-1,1'-binaphthyl, 2-(di-tert-butylphosphino)biphenyl, 2-di-tert-butylphosphino-2'-(N,N-dimethylamino)biphenyl, 2-di-tert-butylphosphino-2'-methylbiphenyl, 2-(dicyclohexylphosphino)biphenyl, 2-dicyclohexylphosphino-2',6'-dimethoxy-1,1'-biphenyl, 2-(dicyclohexylphosphino)-2'-(N,N-dimethylamino)biphenyl, 2-dicyclohexylphosphino-2'-methylbiphenyl, and 2-(dicyclohexylphosphino)-2',4',6'-triisopropyl-1,1'-biphenyl.

Examples of Compound (1) include RhCl(PPh₃)₃ (Wilkinson's complex), RuCl₂(PPh₃)₂, NiCl₂(PPh₃)₂, PdCl₂(PPh₃)₂, CoCl₂(PPh₃)₂, and CoCl(PPh₃)₃. Of these, RhCl(PPh₃)₃ (Wilkinson's complex) is particularly preferably employed.

In the present invention, the Group VIII metal complex (1) is preferably used in an amount of 0.03 to 1 mole equivalent, with respect to the organic halogen compound, more preferably 0.05 to 0.5 mole equivalents.

The Group VIII metal complex (1) may be a commercial product or may be prepared from a corresponding Group VIII metal salt and a neutral ligand.

The hydrogen source (reducing agent) employed in the method of the present invention is a compound represented by M²BHₚR¹_{q} (2-1) or M³(BHₚR¹_{q})₂ (2-2) (hereinafter referred to as Compound (2-1) or Compound (2-2)).
In the above formulas, M² represents an alkali metal atom such as lithium, sodium, or potassium. Of these, lithium and sodium are more preferred, with sodium being particularly preferred. M³ represents an alkaline earth metal atom such as magnesium, calcium, or strontium, or a zinc atom. M³ is preferably calcium or zinc.

R¹ represents a hydrogen atom, a cyano group, a C1-C6 alkoxy group, or a C2-C13 acyloxy group. The C1-C6 alkoxy group may be a linear or branched C1-C6 alkoxy group or a C3-C6 cycloalkyloxy group, and examples include methoxy, ethoxy, and cyclopentyloxy.
Specifically, the C2-C13 acyloxy group may be an alkylcarbonyloxy group, an arylcarbonyloxy group, or an aralkylcarbonyloxy group. Among C2-C13 acyloxy groups, a C2-C7 acyloxy group is preferred. Specific examples of the C2-C7 acyloxy group include an acetyloxy group, a trifluoroacetyloxy group, a benzoyloxy group, and a benzylcarbonyloxy group; and also include an N-isobutyloxycarbonylprolyloxy group and an N-benzyloxycarbonylprolyloxy group.

In the above formulas, p is an integer of 1 to 4, q is an integer of 0 to 3, and the sum of p and q is 4.

Examples of Compound (2-1) or Compound (2-2) include sodium borohydride, lithium borohydride, zinc borohydride, calcium borohydride, sodium cyanoborohydride, and sodium alkoxyborohydride. Of these, sodium borohydride is particularly preferably employed.

Compound (2-1) or Compound (2-2) is preferably employed in an amount of 1.0 to 10.0 mole equivalents with respect to the organic halogen compound, more preferably 1.1 to 3.0 mole equivalents.

No particular limitation is imposed on the reaction solvent employed in the method of the present invention, and any solvent may be used so long as it does not inhibit reactions. Among the solvents, examples of hydrocarbons, which may be aliphatic or aromatic, include n-hexane, n-pentane, benzene, toluene, and xylene. Examples of alcohols include methanol, ethanol, propanol, isopropanol (IPA), n-butanol, and t-butanol. Examples of ethers include diethyl ether, diisopropyl ether (IPE), methyl t-butyl ether (MTBE), tetrahydrofuran (THF), cyclopentyl methyl ether, dimethoxyethane, and 1,4-dioxane. Examples of amides include dimethylformamide (DMF), dimethylacetamide (DMAc), and N-methyl-2-pyrrolidone (NMP). Examples of cyclic ureas include 1,3-dimethyl-2-imidazolidinone (DMI) and 1,3-dimethyl-3,4,5,6-tetrahydro-2(1H)-pyrimidinone (DMPU). Examples of halogenohydrocarbons include chloroform, methylene chloride, and 1,2-dichloroethane (EDC). Other solvents such as water, dimethylsulfoxide (DMSO), sulfolane, acetonitrile, acetic acid esters, and acetone may also be used. These solvents may be used singly or in combination of a plurality of species.

Among these solvents, amides such as dimethylformamide (DMF), N-methyl-2-pyrrolidone (NMP), and dimethylacetamide (DMAc); alcohols such as methanol, ethanol, and isopropanol (IPA); dimethylsulfoxide; ethyl acetate; and tetrahydrofuran (THF) are preferred, with amides such as N-methyl-2-pyrrolidone (NMP) and dimethylacetamide (DMAc) and alcohols such as isopropanol being more preferred, with amides such as N-methyl-2-pyrrolidone (NMP) and dimethylacetamide (DMAc) being particularly preferred.

The solvent is used in a total amount of 1 to 20 (v/w) with respect to the Group VIII metal complex (1), more preferably 3 to 10 (v/w).

The reaction temperature may be 0°C to the boiling point of the solvent employed, and is particularly preferably 50 to 80°C.

According to method of the present invention, an organic halogen compound is hydrodehalogenated. Specifically, a halogen atom of an organic halogen compound is substituted with a hydrogen atom, thereby removing the halogen atom. The halogen atom to be removed is a halogen atom on a carbon atom, and is substituted with a carbon atom constituting an aromatic compound or an aliphatic compound. Therefore, the compounds to which the method of the present invention can be applied are aromatic halogen compounds and aliphatic halogen compounds.
The "aromatic compound," may be an aromatic hydrocarbon compound or an aromatic heterocyclic compound, and may be monocyclic or polycyclic.

Examples of the "monocyclic aromatic compound" include benzene and 5- or 6-membered aromatic heterocyclic compounds. Examples of "5- or 6-membered heterocyclic compounds" include furan, thiophene, pyrrole, pyran, thiopyran, pyridine, thiazole, imidazole, pyrimidine, and 1,3,5-triazine.

Examples of the "polycyclic aromatic compound" include polycyclic aromatic hydrocarbon compounds and polycyclic heteroaromatic compounds. Examples of "polycyclic aromatic hydrocarbon compounds" include biphenyl, triphenyl, naphthalene, indene, anthracene, and phenanthrene, and examples of "polycyclic heteroaromatic compounds" include indole, quinoline, and purine.

The aromatic halogen compound has a basic structure of the aforementioned "aromatic compound" to which one or more halogen atoms have been introduced at substitutable positions. Preferably, 1 to 4 halogen atoms are introduced. Examples of the halogen atom include fluorine, chlorine, bromine, and iodine. Of these, fluorine and chlorine are preferred. When a plurality of halogen species are introduced, fluorine, chlorine, bromine, and iodine may be introduced in any combination. One or more conventionally known functional groups may be introduced.

In the present invention, examples of preferred aromatic halogen compounds include aromatic fluorine compounds and aromatic chlorine compounds, and specific examples include fluorobenzene, difluorobenzene, trifluorobenzene, tetrafluorobenzene, 1-fluoronaphthalene, 2-fluoronaphthalene, 2-fluoropyridine, 3-fluoropyridine, 6-fluoroquinoline, 7-fluoroquinoline, chlorobenzene, dichlorobenzene, trichlorobenzene, tetrachlorobenzene, 1-chloronaphthalene, 2-chloronaphthalene, 2-chloropyridine, 3-chloropyridine, 6-chloroquinoline, and 7-chloroquinoline. From any of the aforementioned halogenobenzene compounds, benzene can be recovered. Similarly, from halogenonaphthalene compounds, halogenopyridine compounds, and halogenoquinoline compounds, naphthalene, pyridine, and quinoline can be recovered.

The "aliphatic hydrocarbon compound," which is a basic structure of the aliphatic halogenohydrocarbon compound, may be a chain hydrocarbon, an alicyclic hydrocarbon, etc. Examples of "the chain hydrocarbon" include a C₁-C₂₀ alkane, a C₂-C₂₀ alkene, and a C₂-C₂₀ alkyne. The "C₁-C₂₀ alkane" is preferably a C₁-C₂₀ alkane, more preferably a C₁-C₆ alkane. No particular limitation is imposed on the alkane, and examples include methane, ethane, propane, butane, pentane, and hexane. The "C₂-C₂₀ alkene" is preferably a C₂-C₂₀ alkene, with a C₂-C₆ alkene being more preferred. No particular limitation is imposed on the alkene, and examples include ethene, propene, and butene. The "C₂-C₂₀ alkyne" is preferably a C₂-C₂₀ alkyne, with a C₂-C₆ alkyne being particularly preferred. No particular limitation is imposed on the alkyne, and examples include acetylene, propyne, and butyne. These hydrocarbons may be linear or branched.

The "alicyclic hydrocarbon" may be a C₃-C₂₀ cycloalkane, a C₃-C₂₀ cycloalkene, etc. The "C₃-C₂₀ cycloalkane" is preferably a C₃-C₁₀ cycloalkane. Examples of the cycloalkane include cyclopropane, cyclobutane, cyclopentane, and cyclohexane. The "C₃-C₂₀ cycloalkene" is preferably a C₃-C₁₀ cycloalkene. No particular limitation is imposed on the cycloalkene, and examples include cyclopropene, cyclobutene, cyclopentene, and cyclohexene.
Notably, when the aliphatic compound has a C-C double or triple bond, the unsaturated bond may be hydrogenated. If hydrodehalogenation is performed while the unsaturated bond is maintained, reaction conditions (e.g., low temperature) must be carefully selected.

The aliphatic hydrocarbon halogen compound has a basic structure of the aforementioned "aliphatic hydrocarbon compound" to which one or more halogen atoms have been introduced at substitutable positions. Preferably, 1 to 4 halogen atoms are introduced. Examples of the halogen atom include fluorine, chlorine, bromine, and iodine. Of these, fluorine and chlorine are preferred. When a plurality of halogen species are introduced, fluorine, chlorine, bromine, and iodine may be introduced in any combination. One or more conventionally known functional groups may be introduced.

In the present invention, examples of preferred aliphatic halogenohydrocarbon compounds include aliphatic fluorohydrocarbon compounds, aliphatic chlorohydrocarbon compounds, and aliphatic chlorofluorohydrocarbon compounds. Specific examples preferred compounds include 1-chloro-2-fluorocyclopropanecarboxylic acid derivatives and 2-chloro-2-fluorocyclopropanecarboxylic acid derivatives. According to the method of the present invention, a cyclopropanecarboxylic acid compound can be recovered.

The present invention will next be described by way of examples, which should not be construed as limiting the invention thereto.

### Examples

### Example 1 Hydrodechlorination of chlorobenzene

Under cooling with ice, sodium borohydride (118 mg) was dissolved in N,N-dimethylacetamide (3 mL) with stirring, and chlorobenzene (234 mg) and RhCl(PPh₃)₃ (192 mg) were added to the solution. The reaction mixture was stirred at 70°C for 3 hours, and then diluted with a mixture of phosphate buffer (pH: 7.0) and acetonitrile (30 : 70) and with 1N hydrochloric acid so that the volume of the resultant mixture was adjusted to 50 mL. The diluted mixture was analyzed through high-performance liquid chromatography [column: YMC Inertosil (4.6 x 150 mm), mobile phase: phosphate buffer (pH: 7.0)/acetonitrile = 30/70, flow rate: 1.0 mL/min, and detection wavelength: 254 nm], and benzene was detected. The yield of benzene, as determined through high-performance liquid chromatography, was 94.9%, confirming that hydrodechlorination was successfully performed.

### Examples 2 to 9 Hydrodechlorination of chlorobenzene

The same reaction procedure as employed in Example 1 was repeated, except that the solvent used and the reaction time were changed. The results are shown in Table 1.

**[Table 1]**

| Example | Solvent | Time (h) | Benzene (%) (determined by HPLC) |
|---|---|---|---|
| 2 | NMP | 22 | 92.3 |
| 3 | DMSO | 22 | 30.9 |
| 4 | MeCN | 22 | 25.9 |
| 5 | MeOH | 22 | 7.7 |
| 6 | EtOH | 22 | 20.2 |
| 7. | i-PrOH | 22 | 83.5 |
| 8 | AcOEt | 22 | 44.1 |
| 9 | THF | 22 | 35.3 |

### Examples 10 to 15 Hydrodechlorination of chlorobenzene

The same reaction procedure as employed in Example 1 was repeated, except that the metal complex used and the reaction time were changed. The results are shown in Table 2.

**[Table 2]**

| Example | Metal complex | Time (h) | Benzene (%) (determined by HPLC) |
|---|---|---|---|
| 10 | RhCl₃ (0.1 mole-eq.) PPh₃ (0.3 mole-eq.) | 20 | 66.4 |
| 11 | RuCl(PPh₃)₃ | 20 | 39.9 |
| 12 | NiCl₂(PPh₃)₂ | 20 | 64.5 |
| 13 | PdCl₂(PPh₃)₂ | 20 | 33.7 |
| 14 | CoCl₂(PPh₃)₂ | 20 | 30.5 |
| 15 | CoCl (PPh₃)₃ | 20 | 5.5 |

### Example 16 Hydrodechlorination of 1,2-dichlorobenzene

Under cooling with ice, sodium borohydride (386 mg) was dissolved in N,N-dimethylacetamide (3 mL) with stirring, and 1,2-dichlorobenzene (500 mg) and RhCl(PPh₃)₃ (315 mg) were added to the solution, followed by stirring at 70°C for 23 hours. The reaction mixture was diluted with a mixture of phosphate buffer (pH: 7.0) and acetonitrile (30 : 70) and with 1N hydrochloric acid so that the volume of the resultant mixture was adjusted to 50 mL. The diluted mixture was analyzed through high-performance liquid chromatography [column: YMC Inertosil (4.6 × 150 mm), mobile phase: phosphate buffer (pH: 7.0)/acetonitrile = 30/70, flow rate: 1.0 mL/min, and detection wavelength: 254 nm], and benzene was detected. The yield of benzene, as determined through high-performance liquid chromatography, was 88.8%, confirming that hydrodechlorination was successfully performed.

### Example 17 Hydrodefluorination of fluorobenzene

Under cooling with ice, sodium borohydride (302 mg) was dissolved in N,N-dimethylacetamide (5 mL) with stirring, and fluorobenzene (512 mg) and RhCl(PPh₃)₃ (493 mg) were added to the solution. The reaction mixture was stirred at 70°C for 96 hours, and then diluted with a mixture of phosphate buffer (pH: 7.0) and acetonitrile (30 : 70) and with 1N hydrochloric acid so that the volume of the resultant mixture was adjusted to 50 mL. The diluted mixture was analyzed through high-performance liquid chromatography [column: YMC Inertosil (4.6 x 150 mm), mobile phase: phosphate buffer (pH: 7.0)/acetonitrile = 30/70, flow rate: 1.0 mL/min, and detection wavelength: 254 nm], and benzene was detected. The yield of benzene, as determined through high-performance liquid chromatography, was 94.8%, confirming that hydrodefluorination was successfully performed.

### Example 18 Hydrodefluorination of 1-fluoronaphthalene

Under cooling with ice, sodium borohydride (194.1 mg) was dissolved in N,N-dimethylacetamide (3 mL) with stirring, and 1-fluoronaphthalene (500 mg) and RhCl(PPh₃)₃ (316.5 mg) were added to the solution, followed by stirring at 70°C for 25 hours. The reaction mixture was diluted with a mixture of phosphate buffer (pH: 7.0) and acetonitrile (50 : 50) and with 1N hydrochloric acid so that the volume of the resultant mixture was adjusted to 50 mL. The diluted mixture was analyzed through high-performance liquid chromatography [column: YMC Inertosil (4.6 × 150 mm), mobile phase: phosphate buffer (pH: 7.0)/acetonitrile = 50/50, flow rate: 1.0 mL/min, and detection wavelength: 254 nm], and naphthalene was detected. The yield of naphthalene, as determined through high-performance liquid chromatography, was 76.9%, confirming that hydrodefluorination was successfully performed.

### Example 19 Hydrodehalogenation of tert-butyl 1-chloro-2-fluorocyclopropanecarboxylate

Under cooling with ice, sodium borohydride (146 mg) was dissolved in N,N-dimethylacetamide (2.5 mL) with stirring, and tert-butyl 1-chloro-2-fluorocyclopropanecarboxylate (500 mg) and RhCl(PPh₃)₃ (476 mg) were added to the solution. The reaction mixture was stirred at 40°C for 22.5 hours, and then diluted with a mixture of phosphate buffer (pH: 7.0) and acetonitrile (50 : 50) and with 5N hydrochloric acid so that the volume of the resultant mixture was adjusted to 50 mL. The diluted mixture was analyzed through high-performance liquid chromatography [column: YMC Inertosil ODS-3 (4.6 x 150 mm), mobile phase: phosphate buffer (pH: 7.0)/acetonitrile = 50/50, flow rate: 1.0 mL/min, and detection wavelength: 220 nm], and tert-butyl cyclopropanecarboxylate was detected.
The yield of tert-butyl cyclopropanecarboxylate, as determined through high-performance liquid chromatography, was 53.1%, confirming that hydrodechlorofluorination was successfully performed.

### Example 20 Hydrodehalogenation of tert-butyl 1-chloro-1-fluorocyclopropanecarboxylate

Under cooling with ice, sodium borohydride (486 mg) was dissolved in N,N-dimethylacetamide (2.5 mL) with stirring, and tert-butyl 1-chloro-1-fluorocyclopropanecarboxylate (500 mg) and RhCl(PPh₃)₃ (476 mg) were added to the solution. The reaction mixture was stirred at 40°C for 22.5 hours, and then diluted with a mixture of phosphate buffer (pH: 7.0) and acetonitrile (50 : 50) and with 5N hydrochloric acid so that the volume of the resultant mixture was adjusted to 50 mL. The diluted mixture was analyzed through high-performance liquid chromatography [column: YMC Inertosil ODS-3 (4.6 × 150 mm), mobile phase: phosphate buffer (pH: 7.0)/acetonitrile = 50/50, flow rate: 1.0 mL/min, and detection wavelength: 220 nm], and the tert-butyl cyclopropanecarboxylate was detected. The yield of tert-butyl cyclopropanecarboxylate, as determined through high-performance liquid chromatography, was 56.3%, confirming that hydrodechlorofluorination was successfully performed.

## Claims

1. A hydrodehalogenation method, comprising treating an organic compound having a halogen atom on a carbon atom thereof in a solvent with a compound represented by formula (2-1) :
M²BHₚR¹_{q} (2-1)
or formula (2-2):
M³ (BHₚ R¹_{q}) ₂ (2-2)
(wherein M² represents an alkali metal atom; M³ represents an alkaline earth metal atom or a zinc atom; R¹ represents a hydrogen atom, a cyano group, a C2-C13 acyloxy group, or a C1-C6 alkoxy group; p is an integer of 1 to 4; q is an integer of 0 to 3; and the sum of p and q is 4) in the presence of a Group VIII metal complex represented by formula (1) :
M¹XₘLₙ (1)
(wherein M¹ represents a Group VIII metal; X represents a halogen atom; L represents a neutral ligand; m is an integer of 1 or 2; n is an integer of 2 or 3; and the sum of m and n is 4).

2. The hydrodehalogenation method as described in claim 1, wherein M¹ is rhodium, palladium, ruthenium, platinum, cobalt, or nickel.

3. The hydrodehalogenation method as described in claim 1, wherein M¹ is rhodium.

4. The hydrodehalogenation method as described in claim 1, wherein X is a chlorine atom.

5. The hydrodehalogenation method as described in claim 1, wherein L is a compound represented by formula (3):
PR²R³R⁴ (3)
(wherein R², R³, and R⁴, which may be the same or different from one another, each represents a C6-C14 aromatic hydrocarbon group or a C1-C30 aliphatic hydrocarbon group).

6. The hydrodehalogenation method as described in claim 1, wherein the compound represented by formula (1) is RhCl(PPh₃)₃.

7. The hydrodehalogenation method as described in any one of claims 1 to 6, wherein M² is a sodium atom.

8. The hydrodehalogenation method as described in any one of claims 1 to 6, wherein the compound represented by formula (2-1) is sodium borohydride.

9. The hydrodehalogenation method as described in any one of claims 1 to 8, wherein the solvent is a single solvent selected from the group consisting of a hydrocarbon-based solvent, a halo-hydrocarbon-based solvent, an alcohol-based solvent, an ether-based solvent, an amide-based solvent, water, a sulfoxide-based solvent, a nitrile, an acetic acid ester-based solvent and a ketone-based solvent, or a mixture of two or more thereof.

10. The hydrodehalogenation method as described in any one of claims 1 to 8, wherein the solvent is an amide-based solvent.

11. The hydrodehalogenation method as described in any one of claims 1 to 8, wherein the solvent is N-methyl-2-pyrrolidone or dimethylacetamide.

12. The hydrodehalogenation method as described in any one of claims 1 to 11, wherein the organic compound having a halogen atom on a carbon atom thereof is an aromatic halogen compound or an aliphatic halogen compound.

13. The hydrodehalogenation method as.described in any one of claims 1 to 11, wherein the halogen atom on a carbon atom is a chlorine atom and/or a fluorine atom.

14. A method for producing a dehalogenated compound, comprising treating an organic compound having a halogen atom on a carbon atom thereof in a solvent with a compound represented by formula (2-1):
M²BHₚ R¹_{q} (2-1)
or formula (2-2):
M³ (BHₚ R¹_{q}) ₂ (2-2)
(wherein M² represents an alkali metal atom; M³ represents an alkaline earth metal atom or a zinc atom; R¹ represents a hydrogen atom, a cyano group, a C2-C13 acyloxy group, or a C1-C6 alkoxy group; p is an integer of 1 to 4; q is an integer of 0 to 3; and the sum of p and q is 4) in the presence of a Group VIII metal complex represented by formula (1) :
M¹XₘLₙ (1)
(wherein M¹ represents a Group VIII metal; X represents a halogen atom; L represents a neutral ligand; m is an integer of 1 or 2; n is an integer of 2 or 3; and the sum of m and n is 4).

15. The production method as described in claim 14, wherein the dehalogenated compound is an aromatic compound or an aliphatic compound.
